# EUROPEAN PATENT APPLICATION

(11) **EP 4 151 213 A1**
(43) Date of publication of application: **22.03.2023**
(21) Application number: 21805235.5
(22) Date of filing: 06.05.2021
(51) Int. Cl.: A61K 31/4709, A61P 25/28

(54) **THERAPEUTIC AGENT FOR MILD COGNITIVE IMPAIRMENT**

(30) Priority: 11.05.2020 JP 2020083163
(71) Applicant: Shimadzu Corporation, Kyoto-shi Kyoto 604-8511 (JP); National Cerebral and Cardiovascular Center, Suita-shi, Osaka 564-8565 (JP)
(72) Inventor: KAWAKAMI Daisuke, Kyoto-shi, Kyoto 604-8511 (JP); IHARA Masafumi, Kishibe-Shimmachi, Suita-shi, Osaka 564-8565 (JP); SAITO Satoshi, Kishibe-Shimmachi, Suita-shi, Osaka 564-8565 (JP)
(74) Representative: Mewburn Ellis LLP
(86) International application number: PCT/JP2021/017368
(87) International publication number: WO 2021/230131

(57) **Abstract**

The present disclosure provides a method of preventing or delaying the progression from mild cognitive impairment (MCI) to dementia or a therapeutic agent therefor.

## Description

### TECHNICAL FIELD

### (Cross-Reference to Related Applications)

This application claims the benefit of Japanese Patent Application No. 2020-83163, filed with the Japan Patent Office on May 11, 2020. The Japanese application is hereby incorporated by reference for all purposes as if the entire set of application documents (specification, claims, drawings, and abstract) were expressly set forth herein.

The present disclosure belongs to the technical field of therapeutic agents for cognitive impairment. The present disclosure relates to a therapeutic agent for mild cognitive impairment containing cilostazol as an active ingredient. In detail, the present disclosure relates to a therapeutic agent for mild cognitive impairment containing cilostazol as an active ingredient, and to dosage and administration of said cilostazol.

### BACKGROUND DISCLOSURE

Mild cognitive impairment (MCI) is a condition that is one step away from dementia. MCI is an intermediate condition between normal and dementia in which memory loss, such as forgetfulness in dementia, is present but the symptoms are still mild. In MCI, regardless of the type of toxic proteins that accumulate, MCI is observed in the early stages, but at such time an irreversible degeneration of the central nervous system does not yet occur in many cases.

Clinically, MCI status is diagnosed on the basis of the following and in other similar situations:
- Whether or not complaints of memory impairment from the individual or his/her family members are acknowledged.
- Whether or not there is objective evidence of impairment in one or more cognitive functions (memory, orientation, etc.).
- Whether or not activities of daily living are normal.
- Whether or not the individual is suffering from dementia.

On the other hand, there are several types of dementia, depending on differences in the sites of brain lesions, etc. Alzheimer's disease, cerebrovascular dementia, frontotemporal dementia, dementia with Lewy body disease, and dementia associated with Parkinson's disease are known. Pathologically irreversible neurodegeneration is often observed in these dementias.

The typical dementia is Alzheimer's type, which accounts for about 50% to 70% of all dementias (Non-Patent Document 1). And even in the stage of MCI, which is one step before becoming Alzheimer's dementia, accumulation of amyloid beta in the brain, the cause of Alzheimer's disease, is recognized as well as it is in Alzheimer's dementia. Therefore, if such MCI is left untreated, Alzheimer's disease is expected to develop within a few years.

While it is difficult to cure Alzheimer's disease completely, in MCI caused by Alzheimer's disease, on the other hand, the onset of dementia can be delayed if an appropriate therapeutic intervention is provided.

Among methods for screening to determine whether MCI is present, MOCA (Montreal Cognitive Assessment), for example, is known. The MOCA consists of testing for visuospatial and executive functions, naming, memory, attention, recitation, word recall, abstract concepts, delayed replay, and orientation. In the MOCA, a score of 25 or less is indicative of MCI, with a sensitivity of 80% to 100%, and specificity of 50% to 87%.

### PRIOR ART

### PATENT DOCUMENT

[Non-Patent Document 1] Blennow K, de Leon MJ, Zetterberg H.: Alzheimer's disease. Lancet. 2006 Jul 29; 368(9533): 387-403

### SUMMARY OF THE DISCLOSURE

### PROBLEM TO BE SOLVED BY THE DISCLOSURE

The present disclosure mainly aims to provide a method of preventing or delaying the progression from mild cognitive impairment (MCI) to dementia or a therapeutic agent therefor. It also aims to provide a method effective for improving cognitive functions in patients with (MCI) or a therapeutic agent therefor.

### MEANS FOR SOLVING THE PROBLEM

The inventors, as a result of intensive studies, have created a new method capable of improving cognitive function through monitoring the effect of cilostazol in the treatment of MCI. Then, the inventors found that the method can solve the above-mentioned problems and they have completed the present disclosure.

The present disclosure, for example, can include the following.

[1] A therapeutic agent for MCI or dementia that is a composition comprising cilostazol or a salt thereof as an active ingredient, and that is either administered to a subject at a first dose of the active ingredient in advance in order to determine a second dose of the active ingredient to be administered to the subject to treat MCI or dementia, or administered to the subject at the second dose of the active ingredient after administration to the subject at the first dose to treat MCI or dementia.
[2] The therapeutic agent for MCI or dementia according to [1] above, wherein the presence amount of cilostazol, OPC-13015, and/or other metabolites in samples derived from the subject is measured after administration of the first dose of the active ingredient to the subject.
[3] The therapeutic agent for MCI or dementia according to [2] above, wherein the presence ratio of cilostazol or OPC-13015 relative to the total presence amount of cilostazol and OPC-13015, or the presence ratio of OPC-13015 relative to the total presence amount of cilostazol, OPC-13015, and OPC-13213 and/or OPC-13217 measured in the sample derived from the subject, after administration of the first dose of the active ingredient to the subject, is calculated.
[4] The therapeutic agent for MCI or dementia according to [3] above, wherein the second dose of the active ingredient is determined within the range of a pharmaceutically acceptable dose of cilostazol based on the said presence ratio of cilostazol or OPC-13015.
[5] The therapeutic agent for MCI or dementia according to [4] above, wherein, taking the said presence ratio of cilostazol or OPC-13015 in an improved/maintained cognitive impairment group that is known to have an improved or maintained cognitive impairment as a reference level, the second dose of the active ingredient is determined at a dose greater than the first dose within the range of a pharmaceutically acceptable dose of cilostazol if the presence ratio of cilostazol in the subject is higher than the reference level of cilostazol or if the presence ratio of OPC-13015 in the subject is lower than the reference level of OPC-13015.
[6] The therapeutic agent for MCI or dementia according to [4] or [5] above, wherein, taking the said presence ratio of cilostazol or OPC-13015 in a worsened cognitive impairment group that is known to have a worsened cognitive impairment as a reference level, the second dose of the active ingredient is determined at a dose greater than the first dose within the range of a pharmaceutically acceptable dose of cilostazol if the presence ratio of cilostazol in the subject is higher than the reference level of cilostazol or if the presence ratio of OPC-13015 in the subject is lower than the reference level of OPC-13015.
[7] The therapeutic agent for MCI or dementia according to any one of [1] to [6] above, wherein the first dose of the active ingredient is 50 mg twice a day, and the second dose is 100 mg twice a day or 150 mg twice a day.
[8] The therapeutic agent for MCI or dementia according to any one of [2] to [7] above, wherein the sampling from the subject is performed within one to seven hours after the first dose of the active ingredient.
[9] The therapeutic agent for MCI or dementia according to any one of [2] to [8] above, wherein the sample is plasma.
[10] The therapeutic agent for MCI or dementia according to [9] above, wherein the sample is pretreated by a protein-removal method using an organic solvent.
[11] The therapeutic agent for MCI or dementia according to any one of [2] to [10] above, wherein the presence amount or ratio is measured by a method selected from the group consisting of LC, ELISA, and LC-MS.
[12] The therapeutic agent for MCI or dementia according to any one of [1] to [11] above, wherein the dementia is Alzheimer's disease.
[13] The therapeutic agent for MCI or dementia according to any one of [1] to [11] above, wherein the MCI is disorientation.
[14] The therapeutic agent for MCI or dementia according to any one of [1] to [13] above, wherein the agent is an orally administered formulation.
[15] The therapeutic agent for MCI or dementia according to any one of [1] to [14] above, wherein the subject is not diagnosed with intermittent claudication or stroke.
[16] A method for determining a second dose of cilostazol for treating MCI impairment or dementia, comprising the following steps 1 to 3:
   1) a step of measuring the presence amounts of cilostazol and OPC-13015 or, in addition to them, the presence amount(s) of OPC-13213 and/or OPC-13217 in a sample derived from a subject who received a first dose of a composition comprising cilostazol or a salt thereof as an active ingredient,
   2) a step of calculating the presence ratio of cilostazol or OPC-13015 relative to the total presence amount of cilostazol and OPC-13015, or the presence ratio of OPC-13015 relative to the total presence amount of cilostazol, OPC-13015, and OPC-13213 and/or OPC-13217, and
   3) a step of determining a second dose of cilostazol within the range of a pharmaceutically acceptable dose of cilostazol based on the above presence ratio.
[17] The method according to [16] above, further comprising a step of comparing the presence ratio of cilostazol or OPC-13015 relative to the total presence amount of cilostazol and OPC-13015, or the presence ratio of OPC-13015 relative to the total presence amount of cilostazol, OPC-13015, and OPC-13213 and/or OPC-13217, in an improved/maintained cognitive impairment group that is known to have an improved or maintained cognitive impairment, with the presence ratio of cilostazol or the presence ratio of OPC-13015 in the subject.
[18] The method according to [16] or [17] above, further comprising a step of comparing the presence ratio of cilostazol or OPC-13015 relative to the total presence amount of cilostazol and OPC-13015, or the presence ratio of OPC-13015 relative to the total presence amount of cilostazol, OPC-13015, and OPC-13213 and/or OPC-13217, in a worsened cognitive impairment group that is known to have a worsened cognitive impairment, with the presence ratio of cilostazol or the presence ratio of OPC-13015 in the subject.
[19] The method according to any one of [16] to [18] above, wherein the sample from the subject is sampled within 1 to 7 hours after the first dose of the active ingredient.
[20] The method according to any one of [16] to [19] above, wherein the sample is plasma.
[21] The method according to [20] above, wherein the sample is pretreated by a protein-removal method using an organic solvent.
[22] The method according to any one of [16] to [21] above, wherein the presence amount or ratio is measured by a method selected from the group consisting of LC, ELISA, and LC-MS.
[23] The method according to any one of [16] to [22] above, wherein the dementia is Alzheimer's disease.
[24] The method according to any one of [16] to [22] above, wherein the MCI is disorientation.
[25] A system for the treatment of MCI or dementia, comprising the therapeutic agent for MCI or dementia according to any one of [1] to [15] above, and an analyzer for measuring the presence amount(s) of cilostazol, OPC-13015, and/or other metabolites in a sample derived from a subject after administration of a first dose of the active ingredient to the subject.
[26] The system for the treatment of MCI or dementia according to [25] above, further comprising a sample pretreatment device.
[27] The system for the treatment of MCI or dementia according to [25] or [26] above, wherein the analyzer is an instrument selected from the group consisting of LC, LC-MS, LC-MS/MS, and an ELISA analyzer.

### EFFECT OF THE DISCLOSURE

The present disclosure enables the prevention or retardation of the progression to dementia in the treatment of mild cognitive impairment (MCI) or dementia. The present disclosure also provides an expectation of improvement of cognitive function in patients with MCI.

### BRIEF DESCRIPTION OF THE DRAWINGS

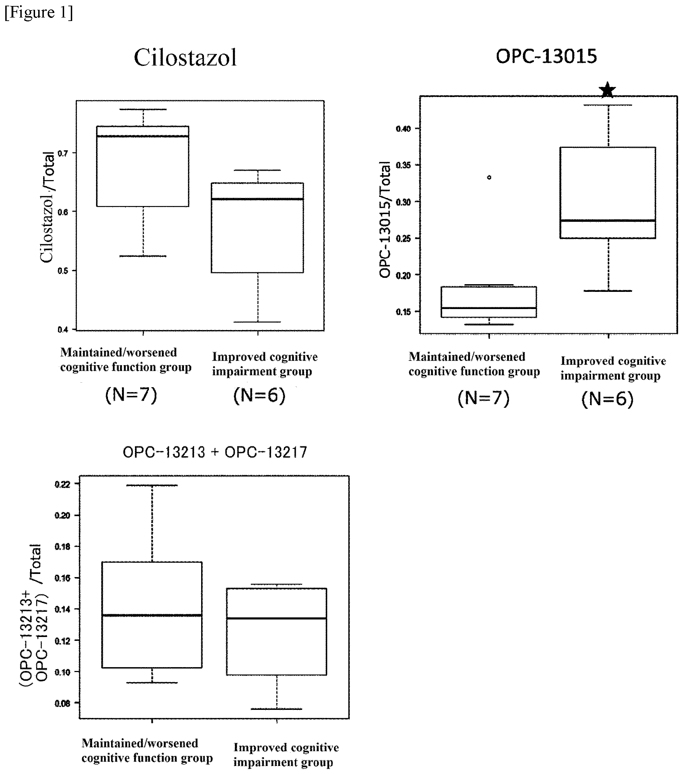

Figure 1 represents the relationship between the plasma concentration ratio of cilostazol or metabolites thereof and improvement in cognitive function. The upper left figure shows the results for cilostazol, the upper right figure shows the results for OPC-13015, and the lower figure shows the results for OPC-13213 + OPC-13217. In each figure, the vertical axis shows the ratio of the plasma concentration of each compound to the total plasma concentration of cilostazol, OPC-13015, and OPC-13213 + OPC-13217.

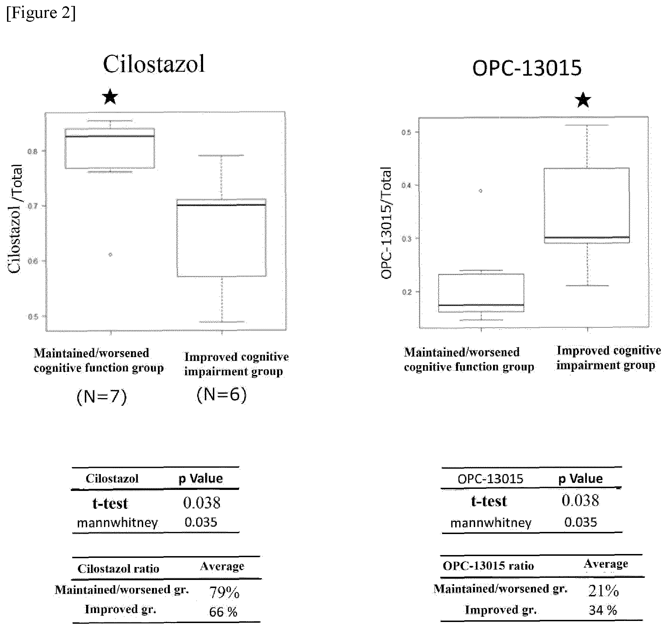

Figure 2 represents the relationship between the plasma concentration ratio of cilostazol or metabolites thereof and improvement in cognitive function. The left figure shows the results for cilostazol, and the right figure shows the results for OPC-13015. In each figure, the vertical axis shows the ratio of the plasma concentration of each compound to the total plasma concentration of cilostazol and OPC-13015.

### EMBODIMENT FOR CARRYING OUT THE PRESENT DISCLOSURE

The following is a detailed description of the present disclosure.

### 1. Definition of Terms

In this description, "cilostazol" is the generic name of 6-[4-(1-cyclohexyl-1H-tetrazol-5-yl)butoxy]-3,4-dihydroquinoline-2(1H)-one and has the following structure of Formula 1. In Japan, Otsuka Pharmaceutical Co. sells it under the trade name "Pletal" (registered trademark) as an antiplatelet agent.

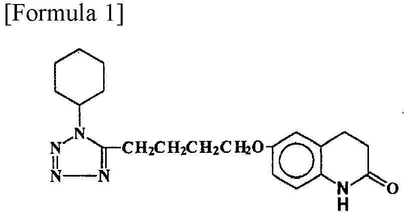

The dosage and administration of the approved cilostazol medicine for adults is usually 100 mg as cilostazol, orally twice daily. This dosage and administration may be adjusted according to age and symptoms. Cilostazol is widely used as an antithrombotic agent, cerebral circulation improving agent, anti-inflammatory agent, anti-ulcer agent, anti-hypertensive agent, and antiasthmatic agent, as well as a phosphodiesterase inhibitor, because it has a phosphodiesterase inhibitory action, an anti-ulcer action, an anti-hypertensive action, and an anti-inflammatory action in addition to a high platelet aggregation inhibitory action.

Cilostazol has also been shown to be effective in preventing the progression of Alzheimer's disease or improving cognitive function (Ihara M, et al.; PLoS One. 2014; 9(2): e89516).

In this description, "OPC-13015," "OPC-13213," "OPC-13217," and "OPC-13326" are metabolites of cilostazol with the following structure. The generic name of OPC-13015 is 6-[4-(1-cyclohexyl-1H-tetrazol-5-yl)butoxy]-2(1H)-quinolinone. The generic name of OPC-13213 is 3,4-dihydro-6-[4-[1-(trans-4-hydroxycyclohexyl)-1H-tetrazol-5-yl)butoxy]-2(1H)-quinolinone. The generic name of OPC-13217 is 3,4-dihydro-6-[4-[1-(cis-4-hydroxycyclohexyl)-1H-tetrazol-5-yl)butoxy]-2(1H)-quinolinone.

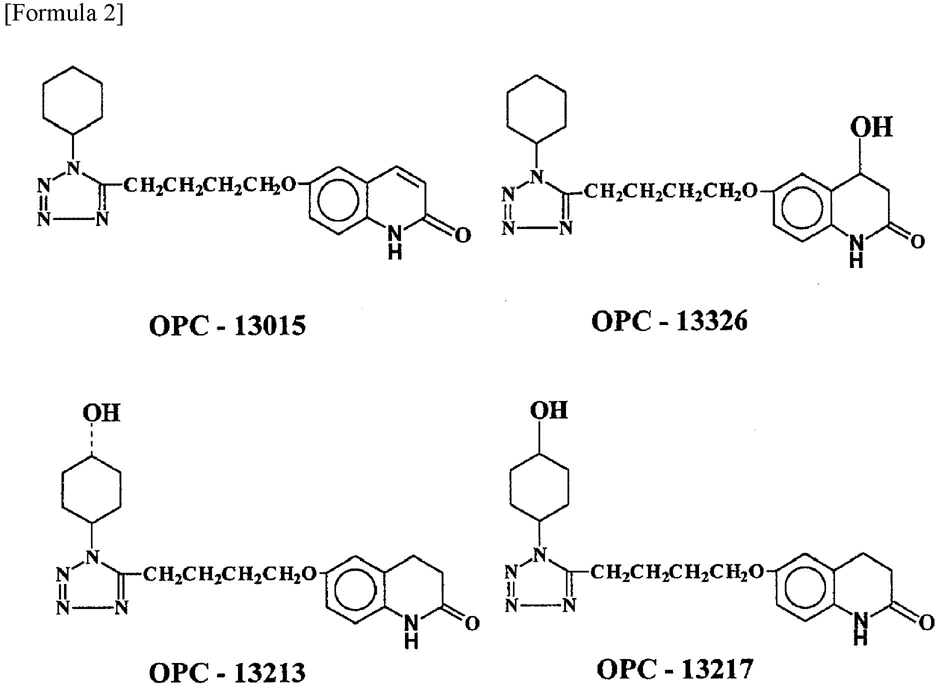

Cilostazol is metabolized in the liver after absorption in the body, and the main metabolites are the four compounds as shown in the above Formula 2 (P. N. V Tata, et al.; J. Pharm. Biomed. Anal.; 18 (1998) 441-451). Of these, OPC-13015 is 3,4-dehydrocilostazol, and OPC-13213 and OPC-13217 are 4'-hydroxycilostazol.

Mild Cognitive Impairment (MCI) in the present disclosure is a pre-dementia condition, as mentioned above, and is mainly accompanied by memory impairment. Whether a patient has MCI is determined, for example, by a psychological screening test of cognitive functioning such as the aforementioned MOCA, Clinical Dementia Rating (CDR), Mini-Mental State Examination (MMSE), the Revised Hasegawa Simplified Intelligence Rating Scale (HDS-R), Clock Drawing Test (CDT), Mini-Cog, and ABC Dementia Scale (ABC-DS).

Of the above, according to the MOCA, a score of 25 or less may be considered MCI. The MMSE is a cognitive function test with a full score of 30 points, and it consists of a total of 11 items, namely time perception, place perception, immediate and delayed playback of three words, calculation, object naming, sentence recitation, three levels of verbal commands, written commands, written writing, and graphic imitation. A score of 23 or less on the MMSE is suspicious for dementia (sensitivity 81%, specificity 89%). A score of 27 or less is suspicious for MCI (sensitivity 45% to 60%, specificity 65% to 90%). While a score between 22 and 26 is considered to be a level of suspected (MCI), a score above 20 is considered to be a level at which the patient can maintain independence (not dementia), and a score of 20 or less is considered to be a level of suspected dementia. A score below 14 is the level requiring guardianship, and a score between 14 and 20 is the level requiring conservatorship or assistance.

The CDR evaluates the severity on a five-point scale for six items: memory, perception, judgment and problem solving, social adjustment, family situation and hobbies/interests, and caregiving status. Considering them together, the severity of the disease is evaluated as either of the following: healthy (CDR: 0), suspected dementia (CDR: 0.5), mild dementia (CDR: 1), moderate dementia (CDR: 2), and severe dementia (CDR: 3).

The HDS-R is a cognitive function test with a full score of 30 points ND consisting of nine items: age, perception, immediate and delayed playback of three words, calculation, number reversal, item recall, and verbal fluency.

The "dementia" can include, for example, Alzheimer's disease (AD), vascular dementia (VD), dementia with Lewy bodies (DLB), frontotemporal lobar degeneration (FTLD), frontotemporal dementia (FTD), semantic dementia (SD), progressive aphasia (PA), and dementia associated with Parkinson's disease. AD is a typical one of these.

The "therapeutic agent" of the present disclosure treats MCI or dementia, but such treatment also includes, for example, prevention and improvement. Therefore, the "therapeutic agent" also includes concepts such as "preventive agent" and "improving agent."

The entire contents of all references cited herein are incorporated herein by reference.

### 1 Therapeutic agent for the treatment of mild cognitive impairment or dementia in accordance with the present disclosure

The therapeutic agent for the treatment of mild cognitive impairment (MCI) or dementia in accordance with the present disclosure (hereinafter referred to as "the present therapeutic agent") is a composition comprising cilostazol or a salt thereof as an active ingredient, which is either administered to a subject at a first dose of the active ingredient prior to determining a second dose of the active ingredient to be administered to the subject to treat MCI or dementia, or administered to the subject at the second dose of the active ingredient after administration to the subject at the first dose to treat MCI or dementia.

Oral dosage forms are preferred for the present therapeutic agent, and solid oral dosage forms such as tablets, capsules, and powders are more preferred, and, unless otherwise specified, the below detailed are oral dosage forms.

The "subject" for the present disclosure is usually a human suffering from MCI or dementia but may also be a person not suffering from MCI or dementia. It may also be a mammal other than humans. It can include, for example, primates (monkeys, marmosets), rodents (mice, rats, guinea pigs, etc.), rabbits, dogs, cats, pigs, cattle, sheep, and horses.

Cilostazol in the present therapeutic agent is preferably in free form, but it may also be a salt of cilostazol. Such salts are not restricted as long as they are pharmaceutically acceptable salts, and they include inorganic salts such as hydrochloride, sulfate, nitrate, phosphate, carbonate, bicarbonate, hydrobromide, and hydroiodide; and organic salts such as formate, acetate, propionate, trifluoroacetate, citrate, lactate, tartrate, oxalate, maleate, fumarate, mandelate, glutarate, malate, benzoate, phthalate, ascorbate, methanesulfonate, ethanesulfonate, isethionate, benzenesulfonate, p-toluenesulfonate, aspartate, glutamate. Such salts also include hydrates and solvates.

In the following, the salts of cilostazol are also referred to simply as cilostazol.

### 1.1 First dose

The first dose for the present disclosure is the dose of cilostazol administered prior to the determination of a second dose (see below) of the active ingredient, cilostazol, to be administered to a subject to treat MCI or dementia.

The first dose may be determined by the physician based on the results of the usual medical diagnosis and, in some cases, the results of appropriate psychological testing. Specifically, for example, 50 mg or 100 mg of cilostazol once or twice a day is appropriate. Depending on the subject's symptoms and other factors, higher or lower doses may be used and more or less frequent administrations may be required.

### 1.2 Second dose

The second dose for the present disclosure is the dose of cilostazol to be determined based on the results after administration of the first dose of cilostazol. The determination of the second dose is usually done by the steps comprising administering a first dose of cilostazol to a subject and then measuring the presence amount of cilostazol, OPC-13015, and/or other metabolites in a sample derived from the subject. Specifically, the second dose is determined within the range of a pharmaceutically acceptable dose of cilostazol on the basis of the presence ratio of cilostazol or OPC-13015 calculated, relative to the total presence amount of cilostazol and OPC-13015 that are measured in a sample derived from a subject after administration of a first dose of the active ingredient to the subject, or the presence ratio of OPC-13015 calculated, relative to the total presence amount of cilostazol, OPC-13015, and OPC-13213 and/or OPC-13217 measured.

Also, taking the presence ratio of cilostazol or OPC-13015 in an improved/maintained cognitive impairment group that is known to have an improved or maintained cognitive impairment as a reference level, the second dose can be determined at a dose greater than the first dose within the range of a pharmaceutically acceptable dose of cilostazol if the presence ratio of cilostazol in the subject is higher than the reference level of cilostazol or if the presence ratio of OPC-13015 in the subject is lower than the reference level of OPC-13015.

In addition to the above reference level, or independently of the above reference level, taking the presence ratio of cilostazol or OPC-13015 in a worsened cognitive impairment group that is known to have a worsened cognitive impairment as a reference level, the second dose can be also determined at a dose greater than the first dose within the range of a pharmaceutically acceptable dose of cilostazol if the presence ratio of cilostazol in the subject is higher than the reference level of cilostazol or if the presence ratio of OPC-13015 in the subject is lower than the reference level of OPC-13015.

Furthermore, in addition to the above reference level, or independently of the above reference level, from the value of cilostazol or OPC-13015 in both the improved/maintained cognitive impairment group that is known to have an improved or maintained cognitive impairment, and the worsened cognitive impairment group that is known to have a worsened cognitive impairment, the respective cutoff values are determined. Then, using the values as the respective reference levels, the second dose can be also determined at a dose greater than the first dose within the range of a pharmaceutically acceptable dose of cilostazol if the presence ratio of cilostazol in the subject is higher than the reference level of cilostazol or if the presence ratio of OPC-13015 in the subject is lower than the reference level of OPC-13015.

The second dose is usually more than the first dose but may be the same or a smaller dose, as the case may be in terms of side effects and efficacy. Specifically, the second dose may vary depending on the presence amounts of cilostazol, OPC-13015, etc., in a sample derived from a subject, and can include, for example, 50 mg, 100 mg, 150 mg, 200 mg, 250 mg, or 300 mg one to three times a day.

Sampling from a subject is suitably performed within one to seven hours after the first dose of cilostazol, preferably within 30 minutes to two hours, but can also be performed at a shorter or longer time as needed.

The sample from a subject is not restricted as long as the presence amount of cilostazol, OPC-13015 and the like in vivo can be measured, but plasma and serum are suitable, and plasma is preferred. When the sample is plasma, the "presence amount" is usually the plasma concentration.

If the sample is a blood sample, it can be a plasma sample obtained after blood-cell components have been separated and removed by centrifugation or other methods. It is preferred that proteins in the plasma sample are removed by pretreatment, using a protein-removal method. The protein-removal method includes a method using insolubilization by protein denaturation, physical removal, etc.

The methods using insolubilization by protein denaturation include, for example, a method using organic solvents, a method using acids, and a method using thermal denaturation (heating, cooling, etc.). Among these methods, the method using organic solvents is preferred.

Polar solvents that are miscible with water are suitable as the organic solvents, and they can include, for example, methanol, ethanol, acetonitrile, and acetone. Among these, methanol and acetonitrile are preferred.

Such protein removal can be performed, for example, using sample aliquoting, reagent aliquoting, agitation, suction filtration, heating, and centrifugation means or methods.

Suitable methods for determining the presence amount or ratio of cilostazol, OPC-13015 in a sample can include, for example, LC (liquid chromatography), LC-MS (Liquid Chromatography-Mass Spectrometry), immunoassay, and ECL (Electrochemiluminescence).

The above LC method includes, for example, the use of various detectors such as UV-visible absorbance detectors and fluorescence detectors to quantify the presence amount or ratio of the substance from the peak area of the chromatogram.

The above LC-MS method includes, for example, a SIM (Selected Ion Monitoring) method using a single mass spectrometry (MS) detector, an MRM (Multiple Reaction Monitoring) method using a tandem mass spectrometry (MS/MS, etc.) detector, or an SRM (Selected Reaction Monitoring). Among these methods, an MRM method (an SRM method) is preferred from the viewpoint of high-sensitivity analysis.

The above immunological analysis method includes, for example, an ELISA (Enzyme-Linked Immunosorbent Assay) method, a FLISA (Fluorescence Linked Immunosorbent Assay) method, an RIA (Radioimmunoassay) method and other immunoassay methods. Among these, an ELISA method and a FLISA method are preferred from the viewpoint of safety and other factors.

Suitable equipment for measuring the presence amount or ratio of cilostazol, OPC-13015, etc., in the sample can include, for example, LC, LC-MS, LC-MS/MS, an ELISA analyzer, and an ECL analyzer, depending on each method mentioned above. Among these, LC-MS and LC-MS/MS are preferred.

### 1.3 Usage

The present therapeutic agent can be used for the treatment of MCI or dementia. MCI and dementia are described above. The present therapeutic agent is especially preferred for use in the treatment of MCI. It is also more preferable to use it for disorientation or a decline of near-term memory among MCI.

By treating MCI, which is a preliminary stage of dementia, with the present therapeutic agent, the progression to dementia can be prevented.

The present therapeutic agent is also preferred for use, for example, in a subject with undiagnosed intermittent claudication or stroke, or in a subject whose stroke is complicated by MCI.

### 1.4 Preparation

For the present therapeutic agent, a commercially available cilostazol formulation can be used as is. Additionally, cilostazol can be used as it is, or as a preparation wherein cilostazol is formulated in the range of 0.01% to 99.5% by weight, preferably in the range of 0.5% to 90% by weight in a pharmaceutically acceptable, non-toxic and inert carrier.

The above carrier can include solid, semi-solid or liquid diluents, fillers and other formulation aids. These can be used singly or in combination of two or more.

The dosage form of the present therapeutic agent may be, for example, in solid or liquid dosage units, and in any forms of oral dosage forms such as powder, capsules, tablets (including orally fast-disintegrating tablets OD), sugar-coated tablets, granules, powder formulations, suspensions, liquids, syrups, elixirs, and troches; and of parenteral dosage forms such as injections, intravenous infusions, and suppositories. It can also be sustained-release formulations. The injections may be a ready-to-use injection kit or an intravenous kit.

Powder can be produced by making cilostazol to an appropriate fineness.

When cilostazol is made into a fine powder, the average particle diameter is usually 10 µm or less, preferably 7 µm or less, and more preferably 5 µm or less. The "average particle diameter" refers to the volume average particle diameter, which is the particle diameter (D_{5 0}, median diameter) at the 50% point of the cumulative distribution from the smallest when measured by the laser-diffraction method. Milling can be dry or wet milling, for example, using a jet mill, a hammer mill, a rotary ball mill, a vibrating ball mill, a shaker mill, a rod mill, a tube mill, etc.

Powder formulations can be made by grinding cilostazol to a suitable fineness and then mixing it with a similarly finely ground pharmaceutical carrier such as starch, or edible carbohydrates such as mannitol. Flavorings, preservatives, dispersants, coloring agents, flavoring agents, etc., can be optionally added.

Capsules can be produced by first filling the powdered form as described above, or the granulated form as described in the section on tablets, into a capsule outer shell such as a gelatin capsule. They can also be produced by mixing the form with lubricants and fluidizers such as colloidal silica, talc, magnesium stearate, calcium stearate, and solid polyethylene glycol in powder and then performing the filling operation. The addition of a disintegrant or solubilizing agent such as carboxymethylcellulose, calcium carboxymethylcellulose, low substituted hydroxypropylcellulose, croscarmellose sodium, sodium carboxymethyl starch, calcium carbonate, or sodium carbonate, can improve the efficacy of the medicine when the capsules are ingested. Alternatively, cilostazol fine powder can be suspended and dispersed in vegetable oil, polyethylene glycol, glycerin, and surfactants, that can then be wrapped in a gelatin sheet to form a soft capsule formulation.

Tablets can be produced by adding excipients to make a powder mixture, granulating or slugging, then adding a disintegrant or lubricant, and then tableting.

Powder mixtures can be produced by mixing suitably powdered substances with the diluents and bases described above. If necessary, binding agents (e.g., sodium carboxymethylcellulose, methylcellulose, hydroxypropylmethylcellulose, gelatin, polyvinylpyrrolidone, and polyvinyl alcohol), dissolution retardants (e.g., paraffin), reabsorbing agents (e.g., quaternary salts), adsorbents (e.g., bentonite, and kaolin), etc. can be added.

The powder mixture can be first wetted with a binding agent, e.g., syrup, starch paste, gum arabic, cellulose solution or polymeric substance solution, then stirred and mixed, after which it can then be dried and crushed to make granules. Instead of granulating the powder in this way, it is also possible to first subject it to a tableting machine and then crush the resulting incomplete form of the slag into granules. To prevent the granules made in this way from sticking to each other, lubricants such as stearic acid, stearate, talc, and mineral oil can be added.

Tablets can also be produced by mixing cilostazol with a flowable inert carrier and then directly tableting without the granulation or slugging process as described above.

The tablets thus produced can be film-coated or sugar-coated. Transparent or translucent protective coatings consisting of an airtight shellac film, sugar or polymeric material coatings, and polishing coatings consisting of waxes can also be used.

Other orally administered formulations, such as liquids, syrups, troches, and elixirs, can also be made in a dosage-unit form so that a certain amount thereof contains a certain amount of cilostazol.

Syrups can be produced by dissolving cilostazol and other ingredients in an appropriate flavored aqueous solution. Elixirs can be produced by using non-toxic alcoholic carriers.

Suspensions can be produced by dispersing cilostazol and other ingredients in a non-toxic carrier. Solubilizers and emulsifiers (e.g., ethoxylated isostearyl alcohols, polyoxyethylene sorbitol esters), preservatives, flavor enhancers (e.g., peppermint oil, saccharin), etc. can be added as needed.

If necessary, dosage-unit formulations for oral administration can be microencapsulated. The formulations can also be coated or embedded in polymers, waxes, etc. to prolong the duration of action or provide sustained release.

Parenteral dosage forms can take the form of liquid dosage units, e.g., solutions or suspensions, intended for subcutaneous, intramuscular or intravenous injection. The parenteral dosage forms can be produced by suspending or dissolving a fixed amount of cilostazol in a non-toxic liquid carrier compatible with the purpose of injection, such as an aqueous or oil-based medium, and then sterilizing the suspension or solution. Non-toxic salts or salt solutions can be added to make the injectable solution isotonic. Stabilizers, preservatives, emulsifiers, etc. can also be added. In a similar way, intravenous drip-infusion formulations can be produced.

Suppositories are produced by dissolving or suspending cilostazol in a water-soluble or insoluble solid of low melting point, such as polyethylene glycol, cocoa fat, semi-synthetic fats [e.g., Witepsol (registered trademark)], higher esters (e.g., myristyl palmitate), or a mixture thereof.

Methods of administration of the present therapeutic agent include, for example, oral, intravenous, portal vein, subcutaneous, drip-infusion, and local administration (e.g., transmucosal, nasal, inhalation, and transdermal administration). The frequency of administration depends on the type of active ingredient, dose, dosage form, patient condition, etc. For example, it can be administered one to several times a day or at intervals of one day to several days.

### 2. Method for determining a second dose of cilostazol for treating MCI or dementia in accordance with the present disclosure

The method of determining a second dose of cilostazol for treating MCI or dementia (hereinafter referred to as "the present disclosure method") includes the following steps 1 to 3:
1) a step of measuring the presence amounts of cilostazol and OPC-13015 or, in addition to them, the presence amount(s) of OPC-13213 and/or OPC-13217 in a sample derived from a subject who received a first dose of a composition comprising cilostazol or a salt thereof as an active ingredient;
2) a step of calculating the presence ratio of cilostazol or OPC-13015 relative to the total presence amount of cilostazol and OPC-13015, or the presence ratio of OPC-13015 relative to the total presence amount of cilostazol, OPC-13015, and OPC-13213 and/or OPC-13217; and
3) a step of determining a second dose of cilostazol within the range of a pharmaceutically acceptable dose of cilostazol based on the above presence ratio.

The meanings of the aforementioned terms such as "MCI," "dementia," "first dose," and "second dose" are synonymous with the foregoing.

The present disclosure method can further comprise a step of comparing the presence ratio of cilostazol or OPC-13015 relative to the total presence amount of cilostazol and OPC-13015, or the presence ratio of OPC-13015 relative to the total presence amount of cilostazol, OPC-13015, and OPC-13213 and/or OPC-13217, in an improved/maintained cognitive impairment group that is known to have an improved or maintained cognitive impairment, with the presence ratio of cilostazol or the presence ratio of OPC-13015 in the subject. By including this step, the second dose can be determined by taking the said presence ratio of cilostazol or OPC-13015 in a cognitive impairment improvement/maintenance group that is known to have improved or maintained cognitive impairment as a reference level, and then determining the second dose at a dose greater than the first dose within the range of a pharmaceutically acceptable dose of cilostazol if the presence ratio of cilostazol in the subject is higher than the reference level of cilostazol or if the presence ratio of OPC-13015 in the subject is lower than the reference level of OPC-13015.

Also, the present disclosure method can further comprise a step of comparing the presence ratio of cilostazol or OPC-13015 relative to the total presence amount of cilostazol and OPC-13015, or the presence ratio of OPC-13015 relative to the total presence amount of cilostazol, OPC-13015, and OPC-13213 and/or OPC-13217, in a worsened cognitive impairment group that is known to have a worsened cognitive impairment, with the presence ratio of cilostazol or the presence ratio of OPC-13015 in the subject. By including this step, the second dose can also be determined by taking the presence ratio of cilostazol or OPC-13015 in a worsened cognitive impairment group that is known to have a worsened cognitive impairment as a reference level, in addition to the above reference level, or independently of the above reference level, and then determining the second dose at a dose greater than the first dose within the range of pharmaceutically acceptable doses of cilostazol if the presence ratio of cilostazol in the subject is higher than the reference level of cilostazol or if the presence ratio of OPC-13015 in the subject is lower than the reference level of OPC-13015.

Cilostazol has been known to be effective in preventing the progression of Alzheimer's disease or improving cognitive functions and has also been known to be effective in treating MCI. However, so far, since a fixed dose of cilostazol has been administered to patients regardless of their conditions, individual differences in the degree of improvement in cognitive function have been observed, and there is the possibility of side effects such as headaches when the dose of cilostazol is increased. The inclusion of these steps enables more accurate determination of the second dose of cilostazol and determination of the appropriate dose of cilostazol for each patient to improve cognitive function.

The subject-derived sample in Step 1 is suitably sampled within one to seven hours after the first dose of cilostazol, preferably within 30 minutes to two hours. It may be sampled in a shorter or longer period if necessary. The sample is not particularly limited as long as it can be used to measure the presence amount of cilostazol, OPC-13015, etc., in vivo, but plasma and serum are appropriate and plasma is preferred. When the sample is plasma, the "presence amount" is usually the plasma concentration.

If the sample is a blood sample, the blood-cell component can be removed by centrifugation or other means to obtain a plasma sample. It is preferable that the plasma sample be pretreated. The pretreatment can be the same as those already described in the description of the present therapeutic agent. It is also the same that the pretreatment by a protein-removal method using an organic solvent such as methanol or acetonitrile is preferred among them. The protein removal can be performed similarly by sample aliquoting, reagent aliquoting, agitation, aspiration filtration, heating, centrifugation, and the like.

Suitable methods for measuring the presence amount or ratio of cilostazol, OPC-13015, etc. in the sample are the same as those described above and include, for example, an LC method, an LC-MS method (e.g., a SIM method, and an MRM method (an SRM method)), an immunological analysis (e.g., an ELISA method, and a FLISA method), and an ECL method.

Suitable instruments for measuring the presence amount or ratio of cilostazol, OPC-13015, etc. in the sample can include, for example, LC, LC-MS, LC-MS/MS, an ELISA analyzer, and an ECL analyzer, depending on each method mentioned above. Among these, LC-MS and LC-MS/MS are preferred.

Although the present disclosure method can be used to determine a second dose of cilostazol to treat MCI or dementia, it is preferred for use in determining the second dose of cilostazol to treat a subject with disorientation, or stroke complicated by MCI, among subjects with MCI. For dementia, the present disclosure method is preferred for use in determining a second dose of cilostazol to treat Alzheimer's disease.

### 3. System for the treatment of MCI or dementia in accordance with the present disclosure

The system for the treatment of MCI or dementia (hereinafter referred to as "the present disclosure system") comprises the present therapeutic agent and an analyzer for measuring the presence amount of cilostazol, OPC-13015, and/or other metabolites in a sample derived from a subject after administration of the first dose of the active ingredient to the subject.

The system can further comprise a sample pretreatment device. The sample pretreatment device can include, for example, a sample dispensing device, a reagent dispensing device, a stirring device, a suction filtration device, a heating device, and a centrifugal device. Also, it can include a sample pretreatment device that integrates the functions of all or at least one of these devices. From the viewpoint of simplicity of operation and reduction of the risk of infection from biological samples, a fully automated sample pretreatment device equipped with each function of sample dispensing, reagent dispensing, stirring, suction filtration or centrifugation, and heating is preferred.

Analytical equipment in the present disclosure system can include, for example, LC, LC-MS, LC-MS/MS, an ELISA analyzer, and an ECL analyzer. Among these, LC-MS and LC-MS/MS are preferred.

### 4. Method of treatment for a patient with MCI or dementia in accordance with the present disclosure

The method of treatment for a patient with MCI or dementia (hereinafter referred to as "the present inventive treatment") comprises a step of administering a second dose of cilostazol or a salt thereof to the patient to whom a first dose of cilostazol or a salt thereof has been administered.

The meanings of the aforementioned terms such as "MCI," "dementia," "first dose," and "second dose" are synonymous with the foregoing.

In the present inventive treatment, further, the total amount of the first dose and the second dose of cilostazol or a salt thereof may be more than 100 mg, which is an oral dose amount per dose of cilostazol or a salt thereof. The present inventive treatment allows treatment with a dose greater than the first dose within the range of a pharmaceutically acceptable dose of cilostazol and may also be expected to prevent or delay the progression to dementia or improve cognitive function in the treatment of MCI or dementia.

In the present inventive treatment, further, the total amount of the first dose and the second dose of cilostazol or a salt thereof may be equal to or less than 200 mg. The present inventive treatment allows treatment with a dose greater than the first dose within the range of a pharmaceutically acceptable dose of cilostazol and may also be expected to prevent or delay the progression to dementia or improve cognitive function in the treatment of MCI or dementia.

The present inventive treatment may further comprise the steps of administering the first dose of cilostazol or a salt thereof to a patient and then collecting a biological sample from the patient before administering the second dose, and detecting OPC-13015 in the biological sample. Additionally, the present inventive treatment may further comprise the steps of administering the first dose of cilostazol or a salt thereof to a patient and then collecting a biological sample from the patient before administering the second dose, and detecting OPC-13213 in the biological sample. Detection of OPC-13015 and/or OPC-13213 may be performed within one to seven hours after the first dose is administered.

The present inventive treatment may further comprise measuring the relative amounts of cilostazol or a salt thereof, OPC-13015, and/or OPC-13213. The present inventive treatment may further comprise calculating the presence ratio of any one of cilostazol, a salt thereof, and OPC-13015, based on the total amount of cilostazol or a salt thereof, OPC-13015, and/or OPC-13213.

### 5. Method of detecting OPC-13015 in a patient with MCI or dementia in accordance with the present disclosure

The method of detecting OPC-13015 in a patient with MCI impairment or dementia in accordance with the present disclosure comprises the steps of detecting cilostazol or a salt thereof in a biological sample taken from a patient with MCI or dementia, and detecting OPC-13015 in the biological sample taken from the patient with MCI or dementia.

The meanings of the aforementioned terms such as "MCI," "dementia," "first dose," and "second dose" are synonymous with the foregoing.

### EXAMPLE

Hereinafter, the present disclosure will be specifically described showing Test Examples. The present disclosure is not limited in any way to the disclosure shown below.

### [Test Example] Effect of cilostazol on MCI

### (1) Subjects

Thirteen subjects with a CDR score of 0.5 who are considered to have MCI. Note that the MOCA scores before the cilostazol administration were less than 25 in all subjects.

### (2) Experimental method

The MOCA psychological test was carried out on each subject prior to the subject taking cilostazol. Then, each subject took either 50 mg or 100 mg tablets of cilostazol twice daily for six months. After six months, the MOCA psychological test was carried out on each subject again. In addition to the MOCA psychological test, blood samples were collected from each subject after six months. The blood samples were pretreated using a sample pretreatment device (CLAM-2000, manufactured by Shimadzu Corporation) to obtain plasma samples. Plasma concentrations of cilostazol and its metabolites in each sample were then measured using an ultra-fast triple quadrupole LC/MS/MS system (LCMS-8040, Shimadzu Corporation).

### (3) Evaluation method

The relationship of the plasma concentration ratios of cilostazol and its metabolites to the difference between the MOCA scores before cilostazol administration (pre-MOCA) and the MOCA scores at six months after administration (post-MOCA) was evaluated.

If the post-MOCA minus pre-MOCA score was 0 or less, the patient was classified into the maintained/worsened cognitive function group. If the post-MOCA minus pre-MOCA score was more than 0, the patient was classified into the improved cognitive function group.

### (4) Results

(4-1) The relationship between the plasma concentration ratio of each of the compounds (cilostazol, OPC-13015, and OPC-13213 + OPC-13217 relative to the total plasma concentration of cilostazol, OPC-13015, and OPC-13213 + OPC-13217 and the maintained/worsened cognitive function group or the improved cognitive function group) was examined. The results are shown in Figure 1. In the figure, asterisks (*) indicate significant differences (p<0.05) in the t-test or Mann-Whitney test.

(4-2) The relationship between the plasma concentration ratio of each of the compounds (cilostazol and OPC-13015 relative to the total plasma concentration of cilostazol and OPC-13015 and the maintained/worsened cognitive function group or the improved cognitive function group) was examined. The results are shown in Figure 2. In the figure, asterisks (*) indicate significant differences (p<0.05) in the t-test or Mann-Whitney test.

(4-3) It is clear from the results shown in the figures that the second dose of cilostazol for a particular subject can be determined through measuring the plasma concentrations of cilostazol, OPC-13015, and other metabolites in a subject after cilostazol administration and then calculating the concentration ratio of cilostazol or OPC-13015 relative to the total concentration of cilostazol and OPC-13015.

## Claims

1. A therapeutic agent for mild cognitive impairment (MCI) or dementia that is a composition comprising cilostazol or a salt thereof as an active ingredient, and that is either administered to a subject at a first dose of the active ingredient in advance in order to determine a second dose of the active ingredient to be administered to the subject to treat MCI or dementia, or administered to the subject at the second dose of the active ingredient after administration to the subject at the first dose to treat MCI or dementia.

2. The therapeutic agent for MCI or dementia according to claim 1, wherein the presence amount of cilostazol, OPC-13015, and/or other metabolites in samples derived from the subject, is measured after administration of the first dose of the active ingredient to the subject.

3. The therapeutic agent for MCI or dementia according to claim 2, wherein the presence ratio of cilostazol or OPC-13015 relative to the total presence amount of cilostazol and OPC-13015, or the presence ratio of OPC-13015 relative to the total presence amount of cilostazol, OPC-13015, and OPC-13213 and/or OPC-13217 measured in the sample derived from the subject, after administration of the first dose of the active ingredient to the subject, is calculated.

4. The therapeutic agent for MCI or dementia according to claim 3, wherein the second dose of the active ingredient is determined within the range of a pharmaceutically acceptable dose of cilostazol based on the said presence ratio of cilostazol or OPC-13015.

5. The therapeutic agent for MCI or dementia according to claim 4, wherein, taking the said presence ratio of cilostazol or OPC-13015 in an improved/maintained cognitive impairment group that is known to have an improved or maintained cognitive impairment as a reference level, the second dose of the active ingredient is determined at a dose greater than the first dose within the range of a pharmaceutically acceptable dose of cilostazol if the presence ratio of cilostazol in the subject is higher than the reference level of cilostazol or if the presence ratio of OPC-13015 in the subject is lower than the reference level of OPC-13015.

6. The therapeutic agent for MCI or dementia according to claim 4 or 5, wherein, taking the said presence ratio of cilostazol or OPC-13015 in a worsened cognitive impairment group that is known to have a worsened cognitive impairment as a reference level, the second dose of the active ingredient is determined at a dose greater than the first dose within the range of a pharmaceutically acceptable dose of cilostazol if the presence ratio of cilostazol in the subject is higher than the reference level of cilostazol or if the presence ratio of OPC-13015 in the subject is lower than the reference level of OPC-13015.

7. The therapeutic agent for MCI or dementia according to claim 1, wherein the first dose of the active ingredient is 50 mg twice a day and the second dose is 100 mg twice a day or 150 mg twice a day.

8. The therapeutic agent for MCI or dementia according to any one of claims 2 to 7, wherein the sampling from the subject is performed within one to seven hours after the first dose of the active ingredient.

9. The therapeutic agent for MCI or dementia according to claim 2, wherein the sample is plasma.

10. The therapeutic agent for MCI or dementia according to claim 9, wherein the sample is pretreated by a protein-removal method using an organic solvent.

11. The therapeutic agent for MCI or dementia according to any one of claims 2 to 10, wherein the presence amount or ratio is measured by a method selected from the group consisting of LC, ELISA, and LC-MS.

12. The therapeutic agent for MCI or dementia according to claim 1, wherein the dementia is Alzheimer's disease.

13. The therapeutic agent for MCI or dementia according to claim 1, wherein the MCI is disorientation.

14. The therapeutic agent for MCI or dementia according to claim 1, wherein the agent is an orally administered formulation.

15. The therapeutic agent for MCI or dementia according to any one of claims 1 to 14, wherein the subject is not diagnosed with intermittent claudication or stroke.

16. A method for determining a second dose of cilostazol for treating MCI or dementia, comprising the following steps 1 to 3:
1) a step of measuring the presence amounts of cilostazol and OPC-13015 or, in addition to them, the presence amount(s) of OPC-13213 and/or OPC-13217 in a sample derived from a subject who received a first dose of a composition comprising cilostazol or a salt thereof as an active ingredient;
2) a step of calculating the presence ratio of cilostazol or OPC-13015 relative to the total presence amount of cilostazol and OPC-13015, or the presence ratio of OPC-13015 relative to the total presence amount of cilostazol, OPC-13015, and OPC-13213 and/or OPC-13217; and
3) a step of determining a second dose of cilostazol within the range of a pharmaceutically acceptable dose of cilostazol based on the above presence ratio.

17. The method according to claim 16, further comprising a step of comparing the presence ratio of cilostazol or OPC-13015 relative to the total presence amount of cilostazol and OPC-13015, or the presence ratio of OPC-13015 relative to the total presence amount of cilostazol, OPC-13015, and OPC-13213 and/or OPC-13217, in an improved/maintained cognitive impairment group that is known to have an improved or maintained cognitive impairment, with the presence ratio of cilostazol or the presence ratio of OPC-13015 in the subject.

18. The method according to claim 16 or 17, further comprising a step of comparing the presence ratio of cilostazol or OPC-13015 relative to the total presence amount of cilostazol and OPC-13015, or the presence ratio of OPC-13015 relative to the total presence amount of cilostazol, OPC-13015, and OPC-13213 and/or OPC-13217, in a worsened cognitive impairment group that is known to have a worsened cognitive impairment, with the presence ratio of cilostazol or the presence ratio of OPC-13015 in the subject.

19. The method according to claim 16, wherein the sample from the subject is sampled within one to seven hours after the first dose of the active ingredient.

20. The method according to claim 16, wherein the sample is plasma.

21. The method according to claim 20, wherein the sample is pretreated by a protein-removal method using an organic solvent.

22. The method according to claim 16, wherein the presence amount or ratio is measured by a method selected from the group consisting of LC, ELISA, and LC-MS.

23. The method according to claim 16, wherein the dementia is Alzheimer's disease.

24. The method according to claim 16, wherein the MCI is disorientation.

25. A system for the treatment of MCI or dementia; comprising the therapeutic agent for MCI or dementia according to any one of claims 1 to 15, and an analyzer for measuring the presence amount(s) of cilostazol, OPC-13015, and/or other metabolites in a sample derived from a subject after administration of a first dose of the active ingredient to the subject.

26. The system for the treatment of MCI or dementia according to claim 25, further comprising a sample pretreatment device.

27. The system for the treatment of MCI or dementia according to claim 25 or 26, wherein the analyzer is an instrument selected from the group consisting of LC, LC-MS, LC-MS/MS, and an ELISA analyzer.
